# EUROPÄISCHE PATENTSCHRIFT

(11) **EP 2 526 411 B1**
(45) Veröffentlichungstag und Bekanntmachung des Hinweises auf die Patenterteilung: **04.04.2018**
(21) Anmeldenummer: 10798339.7
(22) Anmeldetag: 23.12.2010
(51) Int. Cl.: G01N 23/16, G01N 23/18, G01N 33/44, C14B 17/00, G01N 23/04

(54) **PRÜFEINRICHTUNG ZUR BESTIMMUNG DER QUALITÄT VON LEDER**
TEST DEVICE FOR DETERMINING THE QUALITY OF LEATHER
DISPOSITIF D'ESSAI PERMETTANT DE DÉTERMINER LA QUALITÉ DU CUIR

(30) Priorität: 18.01.2010 AT 572010
(43) Veröffentlichungstag der Anmeldung: 28.11.2012
(73) Patentinhaber: Wollsdorf Leder Schmidt & Co. Ges.m.b.H., 8181 Wollsdorf (AT)
(72) Erfinder: KRAUS-GUENTNER, Georg, A-8160 Weiz (AT); KRAUS-GUENTNER, Elisabeth, A-8160 Weiz (AT)
(74) Vertreter: Röggla, Harald
(86) Internationale Anmeldenummer: PCT/EP2010/070654
(87) Internationale Veröffentlichungsnummer: WO 2011/085935

(56) Entgegenhaltungen:
- WO-A1-2008/144717
- DE-A1- 4 216 469
- FR-A1- 2 864 668
- US-A- 6 157 730
- US-A1- 2002 065 611
- US-A1- 2004 066 890

## Beschreibung

Die Erfindung bezieht sich auf eine Prüfeinrichtung zur Bestimmung der Qualität von Leder bei der Lederherstellung, wobei die Prüfeinrichtung zum Prüfen einer Qualitätskategorie des Leders und zum Abgeben eines das Leder bezüglich der Qualitätskategorie kennzeichnenden Qualitätswertes ausgebildet ist, wobei die Prüfeinrichtung Durchleuchtungsmittel zum Prüfen der Homogenität des Leders aufweist, mit denen zumindest Teilbereiche des Leders durchleuchtbar sind und die zum Abgeben von Durchleuchtungsdaten an Analysemittel ausgebildet sind, und dass die Analysemittel zum Vergleichen der Durchleuchtungsdaten mit für Hautschädigungen beziehungsweise Inhomogenitäten in Leder typischen Merkmalsdaten und zum Kategorisieren festgestellter Hautschädigungen geprüfter Teilbereiche des Leders ausgebildet sind und dass vorzugsweise Anzeigemittel zum Anzeigen der kategorisierten Hautschädigungen beziehungsweise des Qualitätswertes des Leders vorzugsweise je Teilbereich des Leders ausgebildet sind.

Bei der Lederherstellung wird in einer Abfolge von mechanischen und chemischen Prozessen aus einer Tierhaut eine fertige Lederhaut erzeugt. Da Leder in einer Vielzahl unterschiedlicher Anwendungen eingesetzt wird, muss vor bzw. nach Prozessschritten während der Lederherstellung die Qualität der Lederhaut bestimmt werden, um festzulegen, für welche Anwendung die Lederhaut oder Teilbereiche der Lederhaut geeignet sind. Der Wert einer Lederhaut ist ebenfalls wesentlich durch die Qualität der fertigen Lederhaut geprägt.

Bei der Qualitätskontrolle von Leder kommen mehrerer unterschiedliche Prüfeinrichtungen zur Prüfung unterschiedlicher Qualitätskriterien zum Einsatz. Mit einem so genannten Taber bzw. Veslic oder Martindale Prüfgerät wird die Abriebfestigkeit des Leders geprüft. Mit einem so genannten Flexometer wird die Knickbeständigkeit des Leders geprüft. Bei einem Flammtest wird die Enflammbarkeit des Leders geprüft. Das Leder wird Licht und UV-Strahlung ausgesetzt, um die Lichtechtheit zu prüfen. Mit einem weiteren Gerät wird eine Farbmessung der Farbe der Oberfläche des Leders durchgeführt. Weiters wird das Leder auf Festigkeit (z.B. Zugfestigkeit, Weiterreisskraft, Dehnbarkeit) geprüft. Abschießend findet eine manuelle und visuelle Endkontrolle statt, bei der im Wesentlichen die Qualität bezüglich Hautschädigungen des Leders als Qualitätskriterium beurteilt wird.

Bei Rindsleder beispielsweise sind eine Vielzahl unterschiedlicher Arten von Hautschädigungen bekannt, die auf Verletzungen, Flechten, Milben, Risse, Gabelstöße und andere Ursachen zurückzuführen sind. Jede Art dieser Hautschädigungen hat eine typische Ausbildung, wobei Gabelstöße beispielsweise einen kleinen runden Umfang und Hautschädigungen durch Mastfalten längliche und parallel verlaufende Umrisse aufweisen.

Für die industrielle Herstellung von Leder wurden Ledermerkmalskataloge erstellt, in denen die einzelnen Arten von Hautschädigungen als Qualitätskriterium definiert und je nach Größe, Umfang und Tiefe der Hautschädigung objektive Qualitätswerte beziehungsweise Qualitätsklassen zugeordnet wurden. Bei der manuellen visuellen Endkontrolle wird jede Lederhaut nach Hautschädigungen abgesucht und Teilbereiche der Lederhaut oder auch der gesamten Lederhaut ein Qualitätswert zugeordnet.

Bei der bekannten Qualitätskontrolle von Leder hat sich als Nachteil erwiesen, dass für die visuelle Endkontrolle nur sehr erfahrene Mitarbeiter eingesetzt werden können, die bereits viele Lederhäute kontrolliert haben. Weiters ist die visuelle Endkontrolle sehr zeitintensiv und beleibt letztendlich eine subjektive Entscheidung, auch wenn durch die Erstellung der Ledermerkmalskataloge objektive Bewertungskriterien geschaffen wurden.

Dokument DE 42 16 469 A1 offenbart eine Prüfeinrichtung zum Prüfen von Leder. Das zu prüfende Leder wird durch aufwändige Manipulation vorerst in den Spalt zwischen Transportwalzen und dann durch die Öffnung der Bestrahlungseinrichtung und unter dem Markiergerät in den Spalt einer Stützwalze und einer Zugwalze eingefädelt. Die Zugwalze weist eine borstenbesetzte Oberfläche auf, um das Leder möglichst ohne Schlupf zu ziehen. Hierdurch kann gegebenenfalls Zug in Vorschubrichtung in dem Leder aufgebaut werden.

Dokument FR 2 864 668 offenbart eine Prüfeinrichtung zur Bestimmung der Qualität von Leder, bei der das Leder auf ein Förderband aufgelegt wird. Ein Streifen streicht mögliche Falten des Leders aus, worauf das Leder durch Auflicht und eine Kamera bezüglich Hautschäden an der Oberfläche begutachtet wird. Die bekannte Prüfeinrichtung weist keine Vorspannmittel zum Vorspannen des Leders während der Prüfung des Leders auf.

Dokument WO 2008/144717 A1 offenbart eine Prüfeinrichtung, die einen Vakuum-Halte-Tisch aufweist, auf dem das zu prüfende Leder aufgelegt werden kann. Der Vakuum-Halte-Tisch saugt das Leder während der Prüfung an.

Dokument US 2004/0066890 A1 offenbart eine Prüfeinrichtung zum Prüfen von auf ein Förderband aufgebrachtem Schüttmaterial. Hierfür weist die Prüfeinrichtung eine Röntgeneinheit auf, die das auf dem Förderband transportierte Schüttmaterial durchleuchtet. Dokument US 6,157,703 offenbart eine Prüfeinrichtung zur Bestimmung der Qualität von Leder, bei der das Leder durch Vorspannmittel in Teilbereichen vorgespannt wird. Das Leder wird durch Rollen den Durchleuchtungsmitteln zugeführt, wobei ein erster Teil der Vorspannmittel durch Rollen gebildet ist, die jeweils schneller als die vorangehenden Rollen angetrieben werden, um das Leder in einer ersten Richtung vorzuspannen. Das Dokument offenbart weiters als zweiten Teil der Vorspannmittel Druckbalken, die das Leder lokal in mehrere Vertiefungen der Rollen drücken, um Teilbereiche des Leders, die sich gerade zwischen den Vertiefungen der Rollen befinden, in der zweiten Richtung vorzuspannen. Diese bekannten Prüfeinrichtungen zur Bestimmung der Qualität von Leder weisen allen den Nachteil auf, dass nicht alle Hautschädigungen zuverlässig erkannt werden können.

Der Erfindung liegt somit die Aufgabe zugrunde, eine objektive Qualitätskontrolle für Leder zu schaffen, bei der zuverlässig alle Hautschädigungen des Leders erkannt werden können. Die Erfindung löst die Aufgabe dadurch, dass Vorspannmittel vorgesehen sind, die zum Vorspannen beziehungsweise Dehnen des Leders in zwei im Wesentlichen senkrecht aufeinander stehenden Richtungen zumindest in für das Durchleuchten vorgesehenen Teilbereichen während des Durchleuchtens ausgebildet sind, wobei die Vorspannmittel durch im Wesentlichen senkrecht zueinander angeordneten Spannbalken gebildet sind

Hierdurch ist der Vorteil erhalten, dass der von der Prüfeinrichtung für die Lederhaut ermittelte Qualitätswert ein nach objektiven Kriterien und durch ein objektives Prüfverfahren ermittelter Qualitätswert ist und somit erstmals die Qualität von Lederhäuten vergleichbar wird. Besonders vorteilhaft ist es bestimmten Teilbereichen der Lederhaut, die mit den Anzeigemittel angezeigt werden können, bestimmte Qualitätswerte zuzuordnen. Diese Qualitätswerte können dann gemeinsam mit dem Schnittmuster für den Zuschnitt von Leder, beispielsweise für die Belederung von Lenkrädern, verarbeitet werden, wodurch eine optimale Nutzung der Lederhaut gewährleistet ist.

Vorteilhaft ist es weiters, wenn Durchleuchtungsmittel eine Röntgenquelle aufweisen, mit der die Lederhäute durchleuchtet werden, da die Durchleuchtung mittels Röntgen eine kostengünstige Ausführungsart ermöglicht. Ebenso könnten aber auch andere, aus der Medizin bekannte Durchleuchtungsverfahren, wie Magentresonanz oder Computertomographie verwendet werden.

Versuche haben gezeigt, dass durch leichtes Vorspannen des Leders Hautschädigungen besser erkannt werden können, weshalb das Vorsehen von Vorspannmitteln bei der Prüfeinrichtung eine bessere Detektierbarkeit der Hautschädigungen gewährleistet.

Es hat sich aus Kosten- und Wartungsgründen als vorteilhaft erwiesen, die Durchleuchtungsmittel als Balken auszubilden, in den das Leder zwischen der Röntgenquelle und dem Röntgendetektor eingespannt wird. Bei einer Ausführungsvariante weist die Prüfeinrichtung Ledertransportmittel zum Transport des Leders durch den Balken und bei einer anderen Ausführungsvariante weisen die Durchleuchtungsmittel Transportmittel auf, um die Durchleuchtungsmittel entlang des Leders zu transportieren.

Besonders vorteilhaft ist es, die Durchleuchtungsmittel ausreichend groß auszubilden, dass eine gesamte Lederhaut bei einem Prüfvorgang geprüft werden kann.

Da Detektoren Pixelfehler verursachen können, hat es sich als vorteilhaft erwiesen, dass die Analysemittel eine Vorverarbeitungsstufe aufweisen, die solche Pixelfehler detektiert und vor der weiteren Verarbeitung der Durchleuchtungsdaten eliminiert. Hierdurch werden wesentlich genauere Qualitätswerte erhalten.

In den Ledermerkmalskatalogen sind die bekannten Arten von Hauschädigungen beschrieben. Es hat sich als vorteilhaft erwiesen elektronisch verarbeitbare Merkmalsdaten zu erstellen und in den Analysemitteln zu speichern, die der visuellen Darstellung der bekannten Arten von Hautschädigungen entsprechen. In den Analysemitteln sind weiters Toleranzbereiche gespeichert, in welchen Größenbereichen diese Merkmalsdaten auftreten können. Basierend auf diesen Merkmalsdaten und Toleranzbereichen sind die Analysemittel zum automatisierten Erkennen von Hautschädigungen anhand der Durchleuchtungsdaten der Lederhaut ausgebildet. Zur besseren Erkennung und weiteren Verarbeitung werden die Intensitätswerte der Bereiche mit Hautschädigungen in den Durchleuchtungsdaten verstärkt, wodurch die Qualitätskontrolle zusätzlich verbessert wird.

Weitere Vorteile der Erfindung werden anhand eines Ausführungsbeispiels nachfolgend beschrieben, auf welches Ausführungsbeispiel die Erfindung aber nicht eingeschränkt ist.
Figur 1 zeigt eine schematische Darstellung eines Rinds, bei dem die Lage typischer Hautschädigungen eingezeichnet ist.
Figur 2 zeigt eine typische Form einer Lederhaut eines Rinds.
Figur 3 zeigt eine Prüfeinrichtung zur Prüfung der Qualität der eingespannten Lederhaut in einer Draufsicht.
Figur 4 zeigt die Prüfeinrichtung gemäß Figur 3 in einer Seitenansicht.
Figur 5 zeigt Anzeigemittel der Prüfeinrichtung mit denen das Ergebnis der Qualitätskontrolle angezeigt wird.

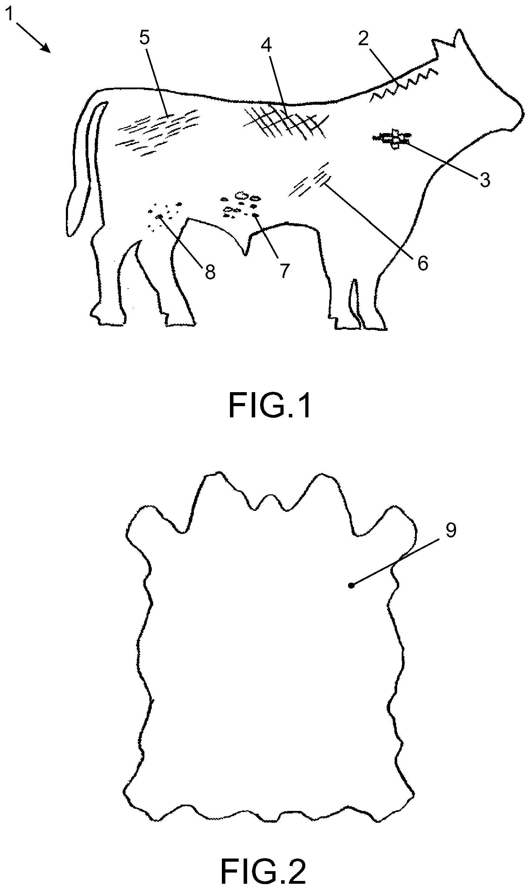

Figur 1 zeigt eine schematische Darstellung eines Rinds 1, wobei die Lage typischer Hautschädigungen eingezeichnet wurde. Am Hals befinden sich durch Heckenrisse und Stacheldrahtrisse bedingte Hautschädigungen 2 und durch Milben bedingte Hautschädigungen 3. Flechten im Fell führen zu Hautschädigungen 4, Dungstellen zu Hautschädigungen 5, Striegelverletzungen zu Hautschädigungen 6, Hornstöße zu Hautschädigungen 7 und Treibstachel sowie Gabelstiche zu Hautschädigungen 8.

Dies ist nur einen Auswahl an Hautschädigungen, die in so genannten Ledermerkmalskatalogen bezüglich ihrer Ausformung (Umfang, typische Größe, Tiefe ins Leder) und Lage kategorisiert wurden.

Eine in Figur 2 dargestellte Lederhaut 9 kann für eine Vielzahl an unterschiedlichen Verwendungszwecken (Ledersofa, Ledermantel, Lederlenkrad,...) weiterverarbeitet werden. Je nach Verwendungszweck, zu erwartender Beanspruchung und Preisklasse des Endproduktes werden Lederhäute unterschiedlicher Qualitätsklassen verarbeitet. Teile der Lederhaut 9 können eine hohe Qualität aufweisen und andere Teile können, beispielsweise durch Hautschäden an der Oberfläche der Lederhaut, eine geringere Qualität aufweisen.

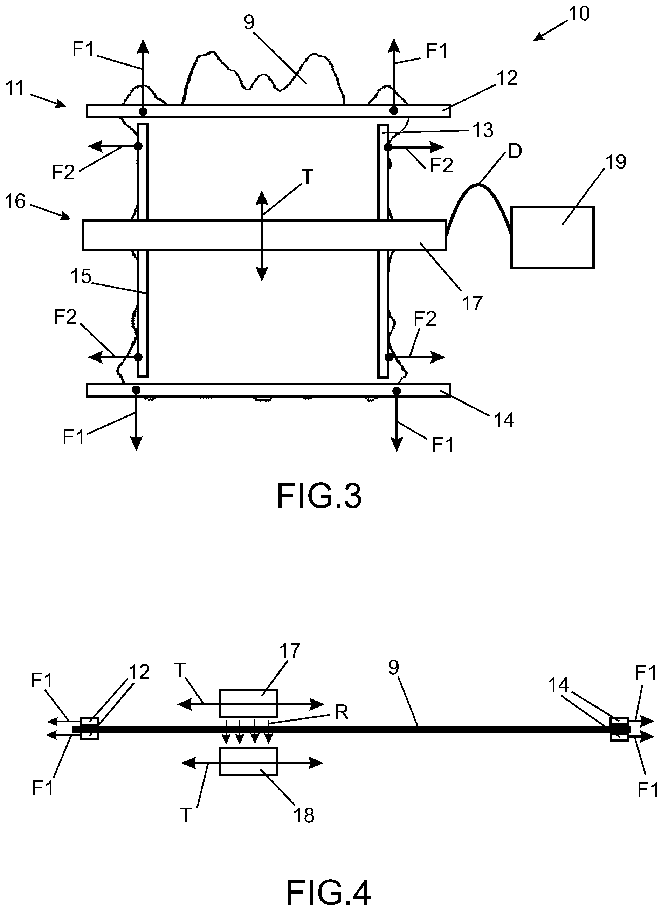

Figur 3 zeigt eine schematische Darstellung einer Prüfeinrichtung 10 zur Prüfung der Qualität der eingespannten Lederhaut 9 in einer Draufsicht. Die Prüfeinrichtung 10 weist Vorspannmittel 11 auf, in die die Lederhaut 9 eingespannt ist. Die Vorspannmittel 11 weisen Spannbalken 12, 13, 14 und 15 auf, die auf in der Figur 3 nicht näher dargestellte Weise, mittels Kräften F1 und F2 auf die Lederhaut 9 einwirken, um diese vorzuspannen. Durch diese Vorspannung wird die Lederhaut um 1% bis 10%, vorzugsweise um 4% bis 7% gedehnt. Dies gewährleistet, dass die Lederhaut 9 eben aufgespannt und gut zu durchleuchten ist.

Die Prüfeinrichtung 10 weist weiters Durchleuchtungsmittel 16 zum Prüfen der Homogenität der Lederhaut 9 auf, mit der praktisch die gesamte Lederhaut 9 durchleuchtbar ist. Die Durchleuchtungsmittel 16 sind durch eine balkenförmig ausgebildete Röntgenquelle 17 und einen in Figur 4 dargestellten ebenfalls balkenförmig ausgebildeten Röntgendetektor 18 gebildet. Die Röntgenquelle 17 gibt Röntgenstrahlen R an den Röntgendetektor 18 ab, wobei die dazwischen befindliche Lederhaut 9 durchleuchtet wird. Ein in den Figuren nicht näher dargestellter Schrittmotor bildet Durchleuchtungsmittel-Transportmittel und ist zum synchronen Transport der Röntgenquelle 17 und des Röntgendetektors 18 in den Richtungen T ausgebildet. Bei einem Durchleuchtungsvorgang transportiert der Schrittmotor die Röntgenquelle 17 und den Röntgendetektor 18 von dem Spannbalken 12 bist zu dem Spannbalken 14, wobei der Röntgendetektor 18 Durchleuchtungsdaten D abgibt.

Die Prüfeinrichtung 10 weist weiters Analysemittel auf, die durch einen Computer 19 gebildet sind. Die von dem Röntgendetektor 18 ermittelten Durchleuchtungsdaten D werden gemeinsam mit Positionsdaten von dem Schrittmotor an den Computer 19 abgegeben. Auf diese Weise sind zu bestimmten Zeiten vom Computer 19 empfangene Durchleuchtungsdaten D bestimmten Teilen der Lederhaut 9 zuordenbar.

Die Analysemittel weisen eine Vorverarbeitungsstufe auf, die von dem Röntgendetektor 18 verursachte Pixelfehler detektiert und Pixelfehlerdaten in den Durchleuchtungsdaten D vor der Weiterverarbeitung durch die Analysemittel eliminiert. Der Röntgendetektor 18 kann beispielsweise an einer bestimmten Stelle einen Defekt aufweisen, weshalb die von dieser Stelle des Röntgendetektors 18 ermittelte Durchleuchtungsdaten D immer einen Maximalwert aufweisen. Dieser Maximalwert wird von der Vorverarbeitungsstufe durch benachbarte Durchleuchtungsdaten D ersetzt. Dem Fachmann sind weitere Verfahren zum Eliminieren von Pixelfehlerdaten bekannt, weshalb hier nicht weiter darauf eingegangen ist.

In dem Computer 19 ist eine Datenbank elektronisch verarbeitbarer Merkmalsdaten von visuellen Darstellungen bekannter Arten von Hautschädigungen von Rindern gespeichert. In diesen Merkmalsdaten sind beispielsweise Durchleuchtungsdaten D gespeichert, die typisch für durch Mastfalten bedingte Hautschädigungen sind. Dem Fachmann im Bereich der Mathematik oder Genanalyse sind Analysemethoden bekannt, bei denen bestimmte zu erwartende Datenstrukturen oder von den erwarteten Datenstrukturen durch vorgegebene Toleranzbereiche abweichende Datenstrukturen in umfangreichen Datenmengen auffindbar und identifizierbar sind. Der Computer 19 analysiert die Durchleuchtungsdaten D, um Merkmalsdaten bestimmter Hautschädigungen zu identifizieren und stellt diese für den Benutzer anschaulich mit einem Monitor 20 des Computers 19 dar. Vorteilhaft ist hierfür die Intensitätswerte der Durchleuchtungsdaten D im Bereich von Hautschädigungen zu verstärken.

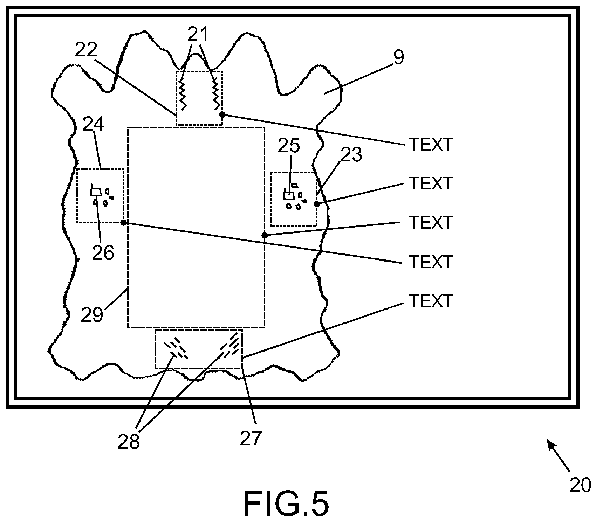

Figur 5 zeigt ein Beispiel, welche Informationen an dem Monitor 20 des Computers 19 dargestellt werden können. Der Umriss der Lederhaut 9 ist dargestellt, wobei von den Durchleuchtungsmitteln 16 ermittelte und von den Analysemitteln des Computers 19 ausgewertete Hautschäden eingezeichnet sind. So sind im Halsbereich der Lederhaut 9 detektierte Risse 21 dargestellt und ein Teilbereich 22 der Lederhaut 9 ist gekennzeichnet. Seitlich am Bildschirm 20 ist in einem Erklärtext der für diesen Teilbereich 22 ermittelte Qualitätswert der Lederhaut und Erläuterungen zu der Art der Hautschädigung angegeben. Teilbereiche 23 und 24 der Lederhaut 9 weisen Hautschäden durch Hornstöße 25 und 26 auf und ein Teilbereich 27 markiert Hautschäden durch Dungstellen 28. Ein Teilbereich 29 kennzeichnet einen Teil der Lederhaut 9, der einen sehr hohen Qualitätswert ohne wesentliche Hautschäden aufweist.

Diese von den Analysemitteln ermittelten und am Monitor 20 dargestellten Teilbereiche der Lederhaut 9 mit unterschiedlichen Qualitätswerten können zur Planung des Einsatzbereiches und des zu legenden Schnittplans für die Weiterverarbeitung verwendet werden, wodurch die Lederhaut 9 optimal verwertet wird.

Gemäß einem weiteren Ausführungsbeispiel der Erfindung wird die Lederhaut in den Vorspannmitteln eingespannt, wobei die Vorspannmittel durch einen Schrittmotor angetrieben Ledertransportmittel zum Transportieren des Leders während des Prüfvorgangs durch die Durchleuchtungsmittel bilden. Der Vorteil bei diesem Ausführungsbeispiel ist, dass die Durchleuchtungsmittel fix montiert sein können und somit weniger fehleranfällig sind.

Es kann erwähnt werden, dass die Prüfung der Qualität des Leders auch unabhängig von der eigentlichen Herstellung des Leders durch eine andere Firma durchgeführt werden kann und der Schutzumfang dieses Patents entsprechend auszulegen ist. Eine erfindungsgemäße Prüfeinrichtung wird in den meisten Fällen Anzeigemittel zur Anzeige der kategorisierten Hautschädigungen aufweisen, es ist aber auch möglich, dass die entsprechenden Daten nicht angezeigt, aber gemeinsam mit der Lederhaut an den weiterverarbeitenden Betrieb zum Zuschneiden der Lederhaut weitergegeben werden.

Es kann erwähnt werden, dass die Durchleuchtungsmittel auch als flächiger Röntgenapparat und flächiger Röntgendetektor ausgebildet sein können, die eine gesamte Lederhaut ohne hierfür nötige Transportmittel durchleuchten. Weiters ist es möglich, dass flächenmäßig relativ kleine Durchleuchtungsmittel die Lederhaut nach einem vorgegebenen Durchleuchtungsschema abfahren und auf diese Weise Durchleuchtungsdaten der gesamten Lederhaut gesammelt werden.

Es kann erwähnt werden, dass die erfindungsgemäße Prüfeinrichtung auch zur Prüfung der Qualität von Lederhäuten von Schweinen oder anderen Tieren verwendet werden kann.

## Patentansprüche

1. Prüfeinrichtung (10) zur Bestimmung der Qualität von Leder (9) bei der Lederherstellung, wobei die Prüfeinrichtung (10) zum Prüfen einer Qualitätskategorie des Leders (9) und zum Abgeben eines das Leder (9) bezüglich der Qualitätskategorie kennzeichnenden Qualitätswertes ausgebildet ist, wobei die Prüfeinrichtung (10) Analysemittel (19) und Durchleuchtungsmittel (16) zum Prüfen der Homogenität des Leders (9) aufweist, mit denen zumindest Teilbereiche (22, 23, 24, 27, 29) des Leders (9) durchleuchtbar sind und die zum Abgeben von Durchleuchtungsdaten (D) an die Analysemittel (19) ausgebildet sind, und dass die Analysemittel (19) zum Vergleichen der Durchleuchtungsdaten (D) mit für Hautschädigungen beziehungsweise Inhomogenitäten in Leder (9) typischen Merkmalsdaten und zum Kategorisieren festgestellter Hautschädigungen geprüfter Teilbereiche (22, 23, 24, 27, 29) des Leders (9) ausgebildet sind und dass vorzugsweise Anzeigemittel (20) zum Anzeigen der kategorisierten Hautschädigungen beziehungsweise des Qualitätswertes des Leders (9) vorzugsweise je Teilbereich (22, 23, 24, 27, 29) des Leders (9) ausgebildet sind, **dadurch gekennzeichnet, dass**
Vorspannmittel (11, 12, 13, 14, 15) vorgesehen sind, die zum Vorspannen beziehungsweise Dehnen des Leders (9) in zwei im Wesentlichen senkrecht aufeinander stehenden Richtungen (F1, F2) zumindest in für das Durchleuchten vorgesehenen Teilbereichen (22, 23, 24, 27, 29) während des Durchleuchtens ausgebildet sind, wobei die Vorspannmittel durch im Wesentlichen senkrecht zueinander angeordneten Spannbalken (11, 12, 13, 14, 15) gebildet sind.

2. Prüfeinrichtung (10) gemäß Anspruch 1, **dadurch gekennzeichnet, dass** die Durchleuchtungsmittel (16) eine Röntgenquelle (17) zum Durchleuchten des Leders (9) mit Röntgenstrahlen (R) und einen Röntgendetektor (18) zum Detektieren der Intensität der durch das Leder (9) abgeschwächten Röntgenstrahlen (R) aufweisen und zum Abgeben entsprechender Durchleuchtungsdaten (D) an die Analysemittel (19) ausgebildet sind.

3. Prüfeinrichtung (10) gemäß Anspruch 1, **dadurch gekennzeichnet, dass** die Vorspannmittel (11, 12, 13, 14, 15) zum Vorspannen beziehungsweise Dehnen des Leders (9) um 1% bis 10%, vorzugsweise um 4% bis 7%, ausgebildet sind.

4. Prüfeinrichtung gemäß einem der vorherigen Ansprüche, **dadurch gekennzeichnet, dass** Ledertransportmittel vorgesehen sind, die zum Transportieren des Leders während des Prüfvorgangs durch die Durchleuchtungsmittel ausgebildet sind.

5. Prüfeinrichtung (10) gemäß einem der Ansprüche 1 bis 3, **dadurch gekennzeichnet, dass** Durchleuchtungsmittel-Transportmittel vorgesehen sind, die zum Transportieren der Durchleuchtungsmittel (16) entlang des fix eingespannten Leders (9) zum Durchleuchten des Leders (9) ausgebildet sind.

6. Prüfeinrichtung (10) gemäß einem der vorherigen Ansprüche, **dadurch gekennzeichnet, dass** die Qualität einer gesamten Lederhaut (9) mit der Prüfeinrichtung (10) bestimmbar ist.

7. Prüfeinrichtung (10) gemäß einem der vorherigen Ansprüche, **dadurch gekennzeichnet, dass** die Analysemittel (19) eine Vorverarbeitungsstufe aufweisen, die vom Röntgendetektor (18) verursachte Pixelfehler detektieren und Pixelfehlerdaten in den Durchleuchtungsdaten (D) vor der weiteren Verarbeitung durch die Analysemittel (19) eliminieren.

8. Prüfeinrichtung (10) gemäß einem der vorherigen Ansprüche, **dadurch gekennzeichnet, dass** die Analysemittel (10) zum Suchen von runde und/oder längliche und/oder ovale Strukturen kennzeichnenden Daten in den Durchleuchtungsdaten (D) ausgebildet sind und diese für die weitere Verarbeitung bezüglich ihrer Intensitätswerte gegenüber den restlichen Durchleuchtungsdaten (D) verstärken.

9. Herstellungsverfahren zur Herstellung von Lederhäuten (9), **dadurch gekennzeichnet, dass** während und/oder nach der Herstellung der fertigen Lederhaut (9) ein Prüfschritt zur Prüfung der Qualität der hergestellten Lederhaut (9) mit einer Prüfeinrichtung (10) gemäß einer der vorstehenden Ansprüche durchgeführt wird.

## Claims

1. A test device (10) for determining the quality of leather (9) in the production of leather, wherein the test device (10) is configured for examining a quality category of the leather (9) and for delivering a quality value characterizing the leather (9) in regard to its quality category, wherein the test device (10) has analysis means (19) and screening means (16) for examining the homogeneity of the leather (9), by means of which at least portions (22, 23, 24, 27, 29) of the leather (9) may be screened and which are configured for delivering screening data (D) to the analysis means (19), and wherein the analysis means (19) are configured for comparing the screening data (D) with feature data typical for hide injuries or inhomogeneities of leather (9) and for classifying determined hide injuries of examined portions (22, 23, 24, 27, 29) of the leather (9), and wherein preferably display means (20) are configured for displaying the categorized hide injuries or quality value, respectively, of the leather (9), preferably per portion (22, 23, 24, 27, 29) of the leather (9),
**characterized in that**, in the screening process, there are provided pretensioning means (11, 12, 13, 14, 15), which are configured for pretensioning or stretching, respectively, the leather (9) in two directions essentially perpendicular to each other (F1, F2) at least in the portions (22, 23, 24, 27, 29) destined for screening,
wherein the pretensioning means are formed by tensioning rods (11, 12, 13, 14, 15) arranged essentially perpendicular to each other.

2. A test device (10) according to claim 1, **characterized in that** the screening means (16) have an X-ray source (17) for screening the leather (9) with X-rays (R) and an X-ray detector (18) for detecting the intensity of the X-rays (R) weakened by the leather (9) and for delivering the corresponding screening data (D) to the analysis means (19).

3. A test device (10) according to claim 1, **characterized in that** the pretensioning means (11, 12, 13, 14, 15) are configured for pretensioning or stretching, preferably, the leather (9) by 1% to 10%, preferably by 4% to 7%.

4. A test device according to any of the above claims, **characterized in that** leather transport means are provided, which are configured for transporting the leather through the screening means during the testing process.

5. A test device (10) according to any of the claims 1 to 3, **characterized in that** transport means for the screening means are provided, which are configured for transporting the screening means (16) alongside the securely clamped leather (9) for screening the leather (9).

6. A test device (10) according to any of the above claims, **characterized in that** the quality of the entire leather hide (9) may be determined by the test device.

7. A test device (10) according to any of the above claims, **characterized in that** the analysis means (19) have a pre-processing step detecting defective pixel caused by the X-ray detector (18) and eliminating defective pixel data in the screening data (D) before these are further processed by the analysis means (19).

8. A test device (10) according to any of the above claims, **characterized in that** the analysis means (10) are configured for detecting data in the screening data (D) characterizing round and/or longitudinal and/or oval structures and for amplifying these in regard to their intensity values compared to the other screening data (D) for further processing.

9. A method for producing leather hides (9), **characterized in that** during and/or after the production of the complete leather hide (9) there will be performed an examination step for examining the quality of the produced leather hide (9) using a test device (10) according to one of the above claims.

## Revendications

1. Dispositif d'essai (10) permettant de déterminer la qualité du cuir (9) lors de la fabrication du cuir, dans lequel le dispositif d'essai (10) est conçu pour contrôler une catégorie de qualité du cuir (9) et pour délivrer une valeur de qualité caractérisant le cuir (9) en ce qui concerne la catégorie de qualité, dans lequel le dispositif d'essai (10) présente des moyens d'analyse (19) et des moyens d'éclairage par transparence (16) pour contrôler l'homogénéité du cuir (9), avec lesquels au moins des régions partielles (22, 23, 24, 27, 29) du cuir (9) peuvent être éclairées par transparence et qui sont conçus pour délivrer des données d'éclairage par transparence (D) aux moyens d'analyse (19), et dans lequel les moyens d'analyse (19) sont conçus pour comparer les données d'éclairage par transparence (D) avec des données de caractéristiques typiques pour des défauts de peau ou des hétérogénéités dans le cuir (9) et pour catégoriser des défauts de peau constatés de régions partielles contrôlées (22, 23, 24, 27, 29) du cuir (9) et dans lequel des moyens d'affichage (20) sont de préférence conçus pour afficher les défauts de peau catégorisés ou la valeur de qualité du cuir (9) de préférence par région partielle (22, 23, 24, 27, 29) du cuir (9), **caractérisé en ce qu'**il est prévu des moyens de précontrainte (11, 12, 13, 14, 15), qui sont conçus pour pré-contraindre ou étirer le cuir (9) dans deux directions (F1, F2) essentiellement perpendiculaires l'une à l'autre au moins dans des régions partielles (22, 23, 24, 27, 29) prévues pour l'éclairage par transparence pendant l'éclairage par transparence, dans lequel les moyens de précontrainte sont formés par des poutres de serrage (11, 12, 13, 14, 15) disposées essentiellement perpendiculairement l'une à l'autre.

2. Dispositif d'essai (10) selon la revendication 1, **caractérisé en ce que** les moyens d'éclairage par transparence (16) présentent une source de rayons X (17) pour l'éclairage par transparence du cuir (9) avec des rayons X (R) et un détecteur de rayons X (18) pour détecter l'intensité des rayons X (R) atténués par le cuir (9) et pour délivrer des données d'éclairage par transparence correspondantes (D) aux moyens d'analyse (19).

3. Dispositif d'essai (10) selon la revendication 1, **caractérisé en ce que** les moyens de précontrainte (11, 12, 13, 14, 15) sont conçus pour pré-contraindre ou étirer le cuir (9) de 1 % à 10 %, de préférence de 4 % à 7 %.

4. Dispositif d'essai (10) selon l'une quelconque des revendications précédentes, **caractérisé en ce qu'**il est prévu des moyens de transport du cuir, qui sont conçus pour transporter le cuir à travers les moyens d'éclairage par transparence pendant l'opération de contrôle.

5. Dispositif d'essai (10) selon l'une quelconque des revendications 1 à 3, **caractérisé en ce qu'**il est prévu des moyens de transport des moyens d'éclairage par transparence, qui sont conçus pour transporter les moyens d'éclairage par transparence (16) le long du cuir fixement tendu (9) pour l'éclairage par transparence du cuir (9).

6. Dispositif d'essai (10) selon l'une quelconque des revendications précédentes, **caractérisé en ce que** la qualité d'un chorion entier (9) peut être déterminée avec le dispositif d'essai (10).

7. Dispositif d'essai (10) selon l'une quelconque des revendications précédentes, **caractérisé en ce que** les moyens d'analyse (19) présentent une étape de prétraitement, qui détecte des défauts de pixels causés par le détecteur de rayons X (18) et qui éliminent des données de défauts de pixels dans les données d'éclairage par transparence (D) avant le traitement ultérieur par les moyens d'analyse (19).

8. Dispositif d'essai (10) selon l'une quelconque des revendications précédentes, **caractérisé en ce que** les moyens d'analyse (10) sont conçus pour rechercher des données caractérisant des structures rondes et/ou allongées et/ou ovales dans les données d'éclairage par transparence (D) et qui les renforcent pour le traitement ultérieur en ce qui concerne leur niveau d'intensité par rapport au reste des données d'éclairage par transparence (D).

9. Procédé de fabrication pour la fabrication de chorions (9), **caractérisé en ce que** l'on exécute, pendant et/ou après la fabrication du chorion terminé (9), une étape de contrôle pour contrôler la qualité du chorion fabriqué (9) avec un dispositif d'essai (10) selon l'une quelconque des revendications précédentes.
